# EUROPEAN PATENT APPLICATION

(11) **EP 1 995 305 A1**
(43) Date of publication of application: **26.11.2008**
(21) Application number: 08009461.8
(22) Date of filing: 23.05.2008
(51) Int. Cl.: C12N 1/14, C12Q 1/02, C12P 1/02

(54) **Fungal biocatalysis process in a culture media containing water miscible ionic liquids**

(30) Priority: 25.05.2007 PT 10375207
(71) Applicant: Instituto de Biologia Experimental e Tecnologia (IBET), 2781-901 Oeiras (PT)
(72) Inventor: Da Costa Silva Pereira, Cristina Maria, 2781-901 Oeiras (PT); N. Rebelo, Luis Paulo, 2781-901 Oeiras (PT); Seddon, K.R., 2781-901 Oeiras (PT)
(74) Representative: Ferreira, Maria Silvina

(57) **Abstract**

The present invention refers to a fungal biocatalysis process, wherein the production ability of the filamentous fungi is augmented by supplementation of the organism culture media with water miscible ionic liquids, which were specifically functionalized as to alter the fungal biocatalysis.

## Description

### Technical Domain of the invention

The present invention refers to a novel bio-catalysis process that explores the ability of certain microorganisms, including filamentous fungi, to grown in a culture media supplemented with a water soluble ionic liquid, which was specifically functionalized, and to, consequently, produce new molecules (metabolites). The organic function introduced in the ionic liquid determines the metabolite diversity that has been synthesized by the fungus.
In this manner, the present invention overlaps the domain of biochemistry, with application in pharmaceutical industry, in waste management industry and in any other processes that depend onto an efficient degradation of residues from food industrial processing.

### Background of the invention

Fungi are a vast group of organisms which are classified in a Kingdom belonging to the Eukaryota domain. This group includes organisms of meaningful dimensions, such as mushrooms, but also microscopic organisms, such as yeasts and the filamentous fungi. Fungi have a vegetative body designated stem or soma and grow as unicellular filamentous microscopic structures called hyphae. The macroscopic net of intertwined and interconnected hyphae is named mycelium, which is normally formed around the substrate (food source) where the food is absorbed. Usually, the most conspicuous fungus part is the fructification bodies or sporangia (the reproducible structures where the spores are formed).

The secondary metabolites are normally produced after the active phase of growth and often possess a complex and rare chemical structure. Some metabolites are common to fungi species belonging to high similar taxa, but others are only observed in few species. This restricted distribution implies that there is not a common function for the secondary metabolites in the fungi. Fungal secondary metabolites undergo a combinatory chemical synthesis and are directly controlled by the substrate used by the fungus during growth.

Nowadays, the use of ionic liquids in whole cell bio-catalysis systems have solely exploited the water immiscible ionic liquids, where the ionic solvent phase is used to remove, by partition from the aqueous phase (i.e. the culture media containing the living organism), a substance which is toxic to the microorganisms, such as phenol or ethanol, as one can observe in Baumann, MD, Daugulis, AJ, and Jessop, PG. Phosphonium ionic liquids for degradation of phenol in a two-phase partitioning bioreactor. Appl. Microbiol. Biotechnol. 2005; 67(1): 131-137; Pfruender, H, Jones, R, and Weuster-Botz, D. Water immiscible ionic liquids as solvents for whole cell biocatalysis. J. Biotechnol. 2006; 124(1): 182-190.

However, in the present invention the microorganism grows directly in a culture media containing the water miscible ionic liquid, resulting in a direct influence of the ionic liquid over the fungus metabolism during growth, influencing the fungal bio-catalysis that, consequently, results in a new profile of fungal metabolites.

In the document Swatloski, R. P.; Rogers, R. D.; Holbrey, J. D. "Dissolution and processing of cellulose using ionic liquids," U.S.10/256,521; PCT/US02/31404; WO03/029329, the use of a specific ionic liquid for solubilising cellulose (water insoluble bio-composite) is disclosed, the fungi's ability to grow within the mixed matrix formed by the ionic liquid and the bio-composite (substrate) not being regarded, which would, concomitantly, dissolve the substrate and control the diversity of synthesized fungal metabolites. In the present invention the ionic liquid solvency ability is exploited for achieving a higher composite biodegradation efficiency partially solubilised by the fungi, such being determined by an enhanced fungus accessibility to the substrate (bioavailability), which emphasizes the potential of the present invention for the synthesis of fungal metabolites formed due to the digestion of extremely recalcitrant and poorly water soluble substrates.

Subsequently, the present invention will allow a new process of fungal biocatalysis that is distinct from all known process in the state of the art, because it uses ionic liquids carrying specific organic functionalities in the ions which constitute the salt, thus determining that the components of a certain reactive mixture react differently when placed in different ionic solvents, i.e., the organic function in the ionic liquid determines the extension and the type of the chemical reaction occurring, resulting in distinct products and in the fungal biocatalysis in an ionic liquid the solvent determines, ubiquitously, the synthesis of new fungal metabolites.

Since ionic liquids have excellent solvency properties, their use in fungal bio-catalysis allows exploiting a broad range of substrates, for example, the water insoluble plant composites which can be use for producing novel, yet uncharacterized, molecules.

### General description of the invention

The present invention refers to a novel process of bio-catalysis using living organisms, such as some filamentous fungi species, that grow in a culture media containing an ionic liquid.

The objective of the invention is to improve the producing ability of the filamentous fungus by providing the culture media with water miscible ionic liquids, which were specifically functionalized so as to alter the fungal catalysis and, consequently, control the production of new molecules.

The organic function introduced in the ionic liquid determines the diversity of the metabolites synthesised by the fungus, such being an exclusive characteristic of each fungal species in a particular substrate.

The present invention may be used to exploit the catalytic capacity of fungi species belonging to all taxa, but it preferentially uses fungi that reproduce asexually, not only due to a simple preparation of spore inoculums, but also because these fungi normally grown well in a submerged culture.

In the present invention the filamentous fungi were preferentially exploited because of all above mentioned proprieties, but also due to the remarkable high diversity of the fungi species, their high degradation ability, and the vast range of complex, structural and chemical, metabolites fungi can synthesize.

Initially the capacity of some fungi isolates (originally isolated amongst the fungi cork colonizing community [1-4]) to degrade efficiently lignin and suberin (which are cork cell walls main constituents) was exploited. This fungal community includes some of the following species: *Penicillium adametzii, Penicillium brevicompactum, Penicillium corylophilum, Penicillium diversum, Penicillium decumbens, Penicillium fenneliae, Penicillium glabrum, Penicillium glandicola, Penicillium hiraymae, Penicillium janczewskii, Penicillium variabile, Penicillium olsonii, Penicillium restrictum, Trichoderma longibrachiatum, Mucor plumbeus, Chrysonilia sitophila* and *Cladosporium herbarum.*

The present invention can use all types of fungi fermentations (continuous, batch, semi-batch, feed-batch, etc), exploiting a vast number of different substrates and environmental conditions.

The initial strategy considered the production of endogenous metabolites synthesized during fungus growth in a poor media supplemented with 1% glucose (minimal media, pH 6) that contains per L of water 0,3 g NaNO₄; 0,05 g KC1; 0,05 g MgSO₄.7H₂O; 0,001 g FeSO₄.7H₂O; 0,01 g ZnSO₄.5H₂O; 0,0005 g CuSO₄.5H₂O and 1 g K₂HPO₄.

The culture media is inoculated with 10⁵ spores per mL and fungi micella growth (either in the presence of NaCl or the ionic liquid) is monitored by measuring the optical density of the culture at 600 nm. The culture extract is collected when the fungi culture is in the stationary growth phase. The extract is clarified by centrifugation (micelle removal) and filtered (0.22 µm) before analysis. The mass spectrometry (MS) profiles (preferentially, Electron Spray Ionization (ESI)) allow a rapid search (fingerprint) of all mass (m/Z) present in the sample (50 to 750 Da). Comparing the fungi metabolites profile induced in the NaCl media with the one induced in the presence of 0,05 M of a specific ionic liquid, the ionic liquid induced novel metabolites (mass) can be identified.

The following table lists some of the ionic liquids which can be used in the present invention.

**Table 1. Ionic liquids that can be used in the present invention. Cations and anions are presented separately.**

| **Name** | **Abbreviated Notations** | **R₁** | **R₂** | **R₃** | **R₄** | **R₅** |
|---|---|---|---|---|---|---|
| ***COMMON CATIONS*** | | | | | | |
| ***Pyridinium*** | | | | | | |
| 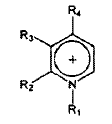 | [CₙPy]⁺ | n-alkyl | *H* | H | H | -- |
| | [Cₙ-3-MePy]⁺ | n-alkyl | *H* | CH₃ | H | -- |
| | [Cₙ-4-MePy]⁺ | n-alkyl | *H* | H | CH₃ | -- |
| ***Bipyridinium*** | | | | | | |
| 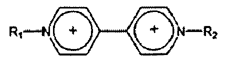 | [R₁, R₂- BPy]⁺ | R₁ | *R₂* | -- | -- | -- |
| ***2,2-Bipyridinium*** | | | | | | |
| 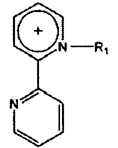 | [Cₙ2,2-BPy]⁺ | n-alkyl | -- | -- | -- | -- |
| ***Pyridazinium*** | | | | | | |
| 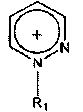 | [R₁-Pydz]⁺ | R₁ | -- | -- | -- | -- |
| ***Pyrimidinium*** | | | | | | |
| 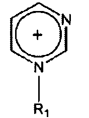 | [R₁-Pym]⁺ | R₁ | -- | -- | -- | -- |
| ***Pyrazinium*** | | | | | | |
| 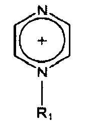 | [R₁-Pyz]⁺ | R₁ | -- | -- | -- | -- |
| ***Cholinium*** | | | | | | |
| 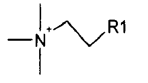 | [Ch]⁺ | OH | -- | -- | -- | -- |
| | [AcCh]⁺ | CH₂COO | -- | -- | -- | -- |
| | [ChOMe]⁺ | OCH₃ | -- | -- | -- | -- |
| ***Imidazolium*** | | | | | | |
| 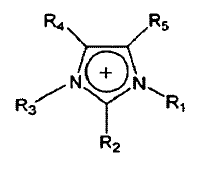 | [mim]⁺ | H | H | CH₃ | H | H |
| | [Cₙmim]⁺ | CH₃ | H | n-alkyl | H | H |
| | [i-Cₙmim]⁺ | CH₃ | H | i-alkyl | H | H |
| | [Cₙim]⁺ | H | H | n-alkyl n≥2 | H | H |
| | [i-Cₙim]⁺ | H | *H* | i-alkyl | H | H |
| | [Cₙ₂Cₙₗlm]⁺ | n₁-alkyl n₁≥2 | *H* | n₂-alkyl n₂≥2 | H | H |
| | [2-MeCₙ₂Cₙ₁im]⁺ | n₁-alkyl n₁≥2 | *CH₃* | n₂-alkyl n₂≥2 | H | H |
| | [2-Memim]⁺ | H | CH₃ | CH₃ | H | H |
| | [2-MeCₙim]⁺ | H | *CH₃* | n-alkyl n≥2 | H | H |
| | [M₅I]⁺ | CH₃ | *CH₃* | CH₃ | CH₃ | CH₃ |
| | [R₃,R₁im]⁺ | R₁ | *H* | R₃ | H | H |
| 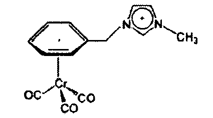 | [Cr(CO)₃(η ⁶-C₆H₅CH₂-mim)]⁺ | -- | -- | -- | -- | -- |
| 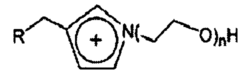 | [PEGₙCₙim]⁺ | *R* = *n-alkyl n*≥*2 PEG* - *poly(ethyleneglycol)* | | | | |
| | [PEGₙmim]⁺ | *R* = *CH₃ PEG* - *poly(ethylene glycol)* | | | | |
| ***Cyclophane-typeimidazolium*** | | | | | | |
| 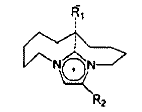 | [Ch₂im]⁺ | H | H | -- | -- | -- |
| | [Ch₃im]⁺ | CH₃ | H | -- | -- | -- |
| ***Pyrrolidinium*** | | | | | | |
| 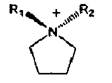 | [P_{n1 ,n2}]⁺ | n₁-alkyl | *n₂*- *alkyl* | -- | -- | -- |
| | [P_{1,n}]⁺ | CH₃ | *n-alkyl* | -- | -- | -- |
| ***Piperidinium*** | | | | | | |
| 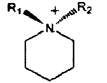 | [Pr_{1,n}]⁺ | CH₃ | *n-alkyl* | -- | -- | -- |
| ***1,4-Dimethylpiperazinium*** | | | | | | |
| 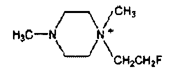 | [(Me)₂-Piz]⁺ | -- | -- | -- | -- | -- |
| ***Pyrazolium*** | | | | | | |
| 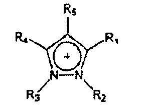 | [R₃,R₂Pyl] + | H | *R₂* | R₃ | H | H |
| | [2-MePyl]⁺ | H | *CH₃* | H | H | H |
| ***Triazolium*** | | | | | | |
| 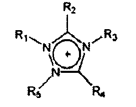 | [R₄-taz₁]⁺ | CH₃ | H | H | R₄ | -- |
| | [1-R₁-2-R2-3- R₃-Taz]⁺ | R₁ | *R₂* | R₃ | H | -- |
| | [3-R₃-Taz₁]⁺ | CH₃ | H | R₃ | H | -- |
| | [1-R₁-3-R₃-Taz]⁺ | R₁ | H | R₃ | H | -- |
| ***Benzotriazolium*** | | | | | | |
| 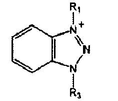 | [CₙC₁BTaz] + | n-alkyl | -- | CH₃ | -- | -- |
| | [R₃- C₁BTaz]⁺ | CH₃ | -- | R₃ | -- | -- |
| ***Oxazolium*** | | | | | | |
| 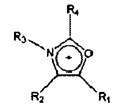 | [R₃O_{X}]⁺ | H | H | R₃ | -- | -- |
| ***Tetrazolium*** | | | | | | |
| 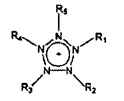 | [1,5-2-NH₂⁻ Ttraz₁]⁺ | NH₂ | *H* | H | CH₃ | NH₂ |
| | [1-R₁-2-R₂-4-R₄-Ttraz₁]⁺ | R₁ | *R₂* | H | R₄ | CH₃ |
| ***Oxazolidinum*** | | | | | | |
| 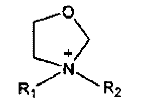 | [Oxd_{1,n}]⁺ | CH₃ | *n-alkyl* | -- | -- | -- |
| | [R₂-Oxd₁]⁺ | CH₃ | *R₂* | -- | -- | -- |
| ***Morpholinium*** | | | | | | |
| 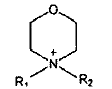 | [Mp_{1,n}]⁺ | CH₃ | *n-alkyl* | -- | -- | -- |
| | [R₂-Mp₁]⁺ | CH₃ | *R₂* | -- | -- | -- |
| ***Thiazolium*** | | | | | | |
| 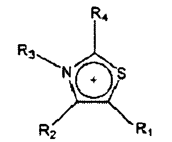 | [R₃th]⁺ | H | *H* | R₃ | H | -- |
| ***Pyrrolinium*** | | | | | | |
| 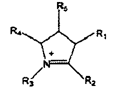 | [R₃-Pyr]⁺ | H | *H* | R₃ | H | H |
| | [2-Me-CₙPyr]⁺ | H | *CH₃* | n-alkyl | H | H |
| ***Ammonium*** | | | | | | |
| 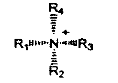 | [N_{n1,n2,n3 ,n4}]⁺ | n₁-alkyl | *n₂*- *alkyl* | n₃-alkyl | n₄-alkyl | -- |
| | [N_{n1 ,n2 ,n3 ,H}]⁺ | n₁-alkyl | *n₂- alkyl* | n₃-alkyl | H | -- |
| ***Phosphonium*** | | | | | | |
| 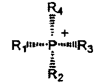 | [P_{n1,n2,n3 ,n4} ]⁺ | n₁-alkyl | *n₂- alkyl* | n₃-alkyl | n₄-alkyl | -- |
| ***Sulfonium*** | | | | | | |
| 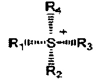 | [S_{n1 ,n2 ,n3} ,ₙ₄]⁺ | n₁-alkyl | *n₂-alky* | n₃-alkyl | n₄-alkyl | -- |
| ***Guanidinium*** | | | | | | |
| 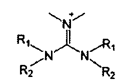 | [G_{n1 ,n2}]⁺ | n₁-alkyl | *n₂- alkyl* | -- | -- | -- |
| 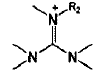 | [GₙG]⁺ | -- | *n₂- alkyl* | -- | -- | -- |
| ***Guanidinium cyclic imidazoline*** | | | | | | |
| 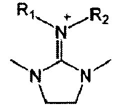 | [Cₙcig₁] ⁺ | CH₃ | *n-alkyl* | *--* | *--* | *--* |
| | [Cₙcig] ⁺ | H | *n-alkyl* | *--* | -- | -- |
| ***Guanidinium cyclic hexahydro-pyrimidine*** | | | | | | |
| 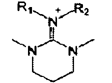 | [Cₙchg₁] ⁺ | CH₃ | *n-alkyl* | *--* | *--* | -- |
| | [Cₙchg] ⁺ | H | *n-alkyl* | *--* | -- | -- |
| ***Guanidinium cyclic tetrahydro-1,3,5-oxadiazine*** | | | | | | |
| 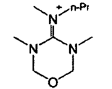 | [C₃ctg₁]⁺ | -- | -- | -- | -- | -- |
| ***Guanidinium cyclic triazoline*** | | | | | | |
| 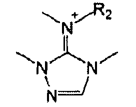 | [Cₙctrag₁] ⁺ | -- | *n-alkyl* | -- | *--* | *--* |
| 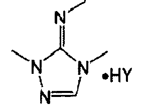 | [Cₙctragh] + | -- | -- | -- | -- | -- |
| ***N-alkyl-isoquinolinium*** | | | | | | |
| 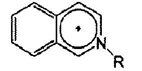 | [CₙIsoq]⁺ | n-alkyl | - | - | - | -- |
| ***CHIRAL IL CATIONS*** | | | | | | |
| ***Chiral imidazolium*** | | | | | | |
| 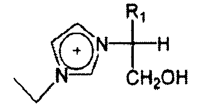 | [Ch2C₂im]⁺ | CH₃ | -- | -- | -- | -- |
| | [Ch3C₂im]⁺ | i-C₂H₅ | -- | -- | -- | -- |
| | [Ch4C₂im]⁺ | i-C₄H₉ | -- | -- | -- | -- |
| 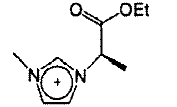 | [Chl-mim]⁺ | -- | -- | -- | -- | -- |
| ***D-1-ethyl-2-(R-phenylethyl)-imidazolium*** | | | | | | |
| 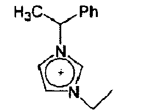 | [Ch5C₂im]⁺ | -- | -- | -- | -- | -- |
| ***Cyclophane-type imidazolium*** | | | | | | |
| 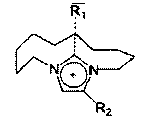 | [Ch4im]⁺ | *CH₃* | *CH₃* | *--* | *--* | *--* |
| ***Chiral oxazolinium*** | | | | | | |
| 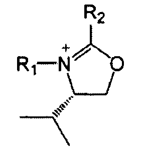 | [2-Et-1-C₅ox]⁺ | *C₅H₁₁* | *C₂H₅* | -- | -- | -- |
| | [2-Et-1-C₁ox]⁺ | *CH₃* | *C₂H₅* | -- | -- | -- |
| 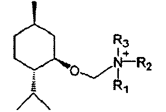 | [Mym-N_{n1,n2,n3}]⁺ | *n-alkyl* | *n-alkyl* | n-alkyl | -- | -- |
| 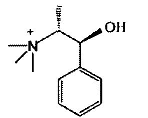 | [Chc4]⁺ | -- | -- | -- | -- | -- |
| 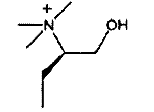 | [Chc5]⁺ | -- | -- | -- | -- | -- |
| 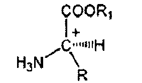 | [ABCCₙ] ⁺ | *n-alkyl* | *H* | [ABC], wherein A, B and C are the three-letter symbols for amino acids | | |
| 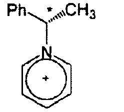 | [Ch5-Py]⁺ | -- | -- | -- | -- | -- |
| 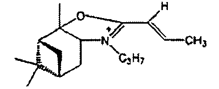 | [Chc8]⁺ | -- | -- | -- | -- | -- |
| ***IL DICATIONS*** | | | | | | |
| ***α, ω-diimidazolium alkylene*** | | | | | | |
| 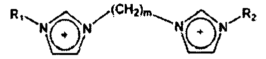 | [Cₘ(Cₙim)₂]⁺ | *n-alkyl* | *n-alkyl* | -- | -- | -- |
| ***1,1-Methylene-4,4-dialkylbis(1,2,4-triazolium)*** | | | | | | |
| 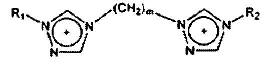 | [Cₘ(CₙTaz)₂] 2+ | *n-alkyl* | *n-alkyl* | -- | -- | -- |
| ***IL ZWITTERIONS*** | | | | | | |
| 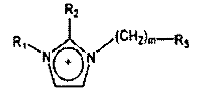 | [Z1im]⁺, m=3 | *C₂H₅* | *H* | SO₃⁻ | -- | -- |
| | [Z2im]⁺, m=3 | *Ethylene* | *H* | SO₃- | -- | -- |
| | [Z3im]⁺, m=2 | *C₂H₅* | *H* | SO₂-N⁻-CH₂CF₃ | -- | -- |
| | [Z4im]⁺, m=3 | *C₂H₅* | *H* | SO₂-N⁻-CH₂CF₃ | -- | -- |
| | [Z5im]⁺, m=2 | *CH₃* | *CH₃* | SO₂-N⁻-CH₂CF₃ | -- | -- |

**R** - functional groups such as amines and amides, ether, alcohol, carboxylic, urea and thiourea groups, phosphine, or nitrile functionalities, N-butyronitrile, fluorous chains, thyol, and alkyne.

| | Anions | Chemical Structure |
|---|---|---|
| [AlY₄]⁻ Y = Cl, Br, I | Aluminium halide | 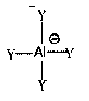 |
| [AuCl₄]⁻ | Tetrachloroaurate | 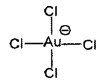 |
| [FeCl₄]⁻ | Tetrachloroferrate | 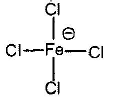 |
| [PdCl₄]⁻ | Tetrachloropalladate | 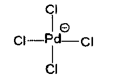 |
| [ClO₄]⁻ | Perchlorate | 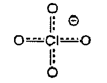 |
| [HBr₂]⁻ | Dibromohydrogenate(I) | H-Br-Br^{⊖} |
| [LA]⁻ | Lactate | 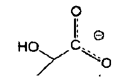 |
| [NO₃]⁻ | Nitrate | 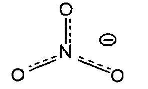 |
| [NO₂]⁻ | Nitrite | 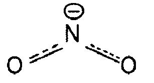 |
| [DCA]⁻ | Dicyanamide | 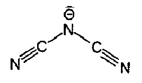 |
| [SCN]⁻ | Thiocyanate | N≡C-S^{⊖} |
| [C(CN)₃]⁻ | Tricyanomethanide | 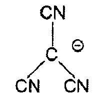 |
| [NfO]⁻ | Nonaflate | 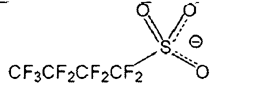 |
| [C₁SO₄]⁻ | Methylsulfate | 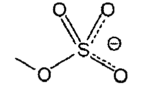 |
| [C₂SO₄]⁻ | Ethylsulfate | 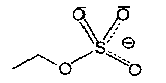 |
| [i-C₄SO₄]⁻ | Isobutylsulfate | 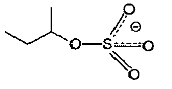 |
| [C₈SO₃]⁻ | Octylsulfate | 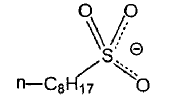 |
| [AcO]⁻ | Acetate | 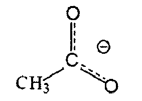 |
| [HSO₄]⁻ | Hydrogen sulfate | 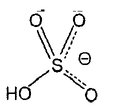 |
| [TA]⁻ = [TFA]⁻ | Trifluoroacetate | 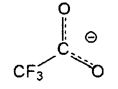 |
| [Tf₂N]⁻ | Bis((trifluoromethyl)sulfonyl)imide | 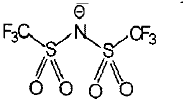 |
| [BETI]⁻ | Bis(perfluoroethylsulfonyl)imide | 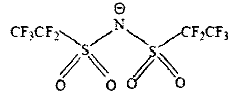 |
| [Me]⁻ | Tris(trifluoromethylsulfonyl)methid | 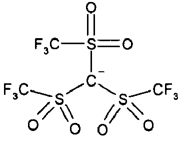 |
| [HB]⁻ | Heptafluorobutanoates | 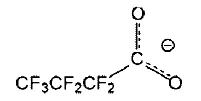 |
| [CB₁₁H₁₂]⁻ | Carborane | 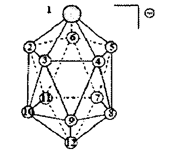 |
| [1-R-CB₁₁H₁₁]⁻ R = C₁ a C₄ | 1-Alkylcarborane | 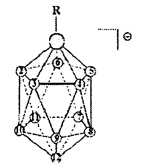 |
| [TfO]⁻ | Trifluoromethanesulphonate, Triflate | 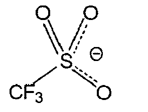 |
| [MeSO₄]⁻ | Mesylate | 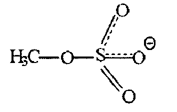 |
| [Sac]⁻ | Saccharinate | 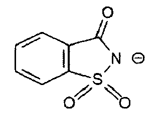 |
| [Ace]⁻ | Acesulfate | 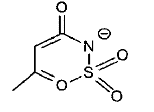 |
| [Tos]⁻ | Tosylate | 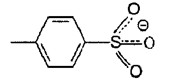 |
| [TTA]⁻ | 2,2,2- -N-(trifluoromethyl sulfonyl)-acetamide | 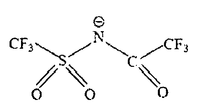 |
| [MeOEtSO₄]⁻ | Methoxyethylsulfate | 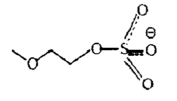 |
| [EtOEtSO₄]⁻ | Ethoxyethylsulfate | 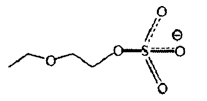 |
| [ACESO]⁻ | 2-[(2-acetamido)amino]ethanesulfate | 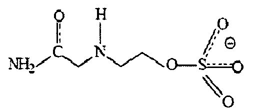 |
| [ACES]⁻ | 2-[(2-acetamido)amino]ethanesulfonate | 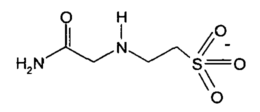 |
| [BESO]⁻ | 2-[Bis(2-hydroxyethyl)amino]ethanesulfate | 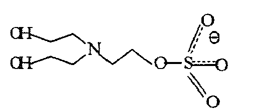 |
| [BES]⁻ | 2-[Bis(2-hydroxyethyl)amino]ethanesulfonate | 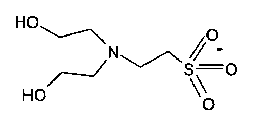 |
| [TAPSO]⁻ | 3-{[Tris(hydroxymethyl)methyl] amino}-1-propanesulfate | 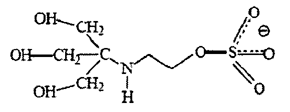 |
| [TAPS]- | 3-{[Tris(hydroxymethyl)methyl] amino}-1-propanesuifonate | 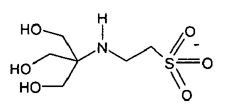 |
| [CHES]⁻ | 2-(Cyclohexylamino)ethanesulfonate | 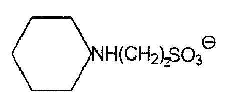 |
| [MOPSO]⁻ | 2-Hydroxy-4-morpholinopropane sulfonate | 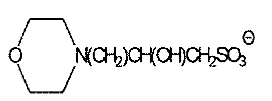 |
| [MES]⁻ | 4-Morpholinoethanesulfonate | 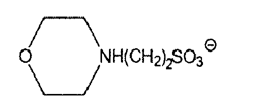 |
| [MOPS]⁻ | 4-Morpholinopropanesulfonate | 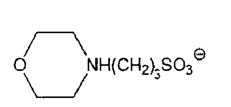 |
| [CAPS]⁻ | 3-(Cyclohexylamino)-1-propanesulfonate | 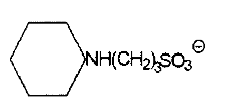 |
| [PF₆]⁻ | Hexafluorophosphate | 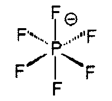 |
| [AsF₆]⁻ | Hexafluoroarsenate | 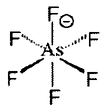 |
| [NbF₆]⁻ | Hexafluoroniobate | 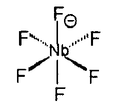 |
| [TaF₆]⁻ | Hexafluorotantalate | 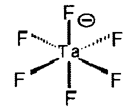 |
| [BF₄]⁻ | Tetrafluoroborate | 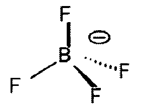 |
| [BPh₄]⁻ | Tetraphenylborate | 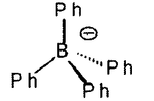 |
| [Barf]⁻ | Tetrakis[p-dimethyl(1H,1H,2H,2 H-perfluorooctyl)silylphenyl]-borate | 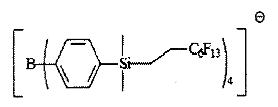 |
| [Y]⁻ | Halogen anions | Y=Cl^{⊖}, Br^{⊖}, I^{⊖} |
| [DOC]⁻ | Docusate | 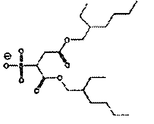 |
| [dtc]⁻ | Diethyldithiocarbamate | 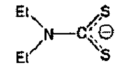 |
| [dtmn]²⁻ | Dithiomaleonitrile | 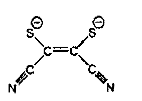 |
| [K-salt]²⁻ | Nitrodithioacetate | 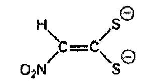 |
| [N(NO₂)₂]⁻ | Dinitramide | 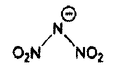 |
| [CuPc(SO₃)₄ ]⁴⁻ | Copper phtalocyaninetetrasulfonate | 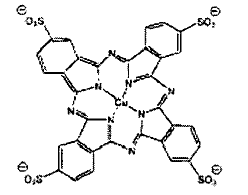 |
| [Trl]⁻ | 3,5-Dinitro-1,2,4-triazolate | 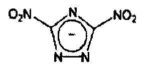 |
| [Dni]⁻ | 4,5-Dinitroimidazolate | 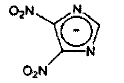 |
| [Ntl]⁻ | 5-Nitrotetrazolate | 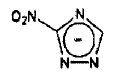 |
| [DBS]⁻ | Dodecylbenzene-sulfonate | 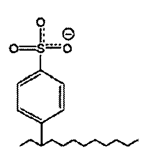 |
| [WOF₅]⁻ | Oxypentafluorotungstate | 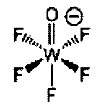 |

The selection of the ionic solvents was rigorously elaborated so as to favor solvent formulations with non-toxic ions and allow the data integration to separately screen the cause-effect of each cation and anion, for e.g. ionic liquids containing the same cations and different anions (and vice versa) are simultaneously tested. The following table shows the cations and the anions initially tested, but the present invention can be used with any water miscible ionic liquid (even when very poorly water soluble).

The concentration of the ionic liquid in the fungi growth media varies from infinite dilution to 10 M, the maximum salt concentration being determined by the species tolerance to the liquid salt in the culture medium. Some fungi species were isolated in environments where the salt concentration (sodium chloride) is extremely high, for e.g. *Eurotium herbariorum* was isolated from the Dead Sea where salt concentration is of 340 g/L (~6M) [5].

The fungal species existing in extreme environments, containing high salt concentrations and low water activity, are adapted fungi species and are not true halophilic or halotolerant species [5, 6], meaning that fungi species occurring in common environments can be gradually adapted to extreme conditions, exploiting continuous cycles of fungi sporulation / germination in increasing concentrations of ionic liquids.

Preliminary results indicated that at concentration levels of 0,05M, about 10% of the ionic liquids tested inhibit spore germination. However, each fungal isolated tested has a particular growth partner in the ionic liquid containing culture media, being impossible to establish any correlation between the fungi species or taxa and its growth behavior in a specific ionic liquid.

In the cases where the fungal spores germinate and mycelium is formed, higher ionic liquid concentrations reduce the culture growth rate (or increase the lag phase extension) without compromising fungi ability to form metabolites.

In the cases where fungi growth is observed, increasing the hydrophobic character of the ionic solvent leads to augmented fungal metabolites diversity.

So being, water miscible functionalized ionic liquids are used to supplement the growth media of microorganisms, being the final concentration in the culture media such that the growth of the selected organism is allowed.

The ionic liquids are totally constituted by ions and the most common formulation for these solvents contains an organic cation and an organic or inorganic anion, whose effect in the fungal culture depends on the cations and anions used, thus it depends mostly on the organic functionalities of the ions (which also determine the solvent toxicity to the living organism).

The ionic liquids (by definition salts which are liquid at room or nearby temperature) carry different organic functionalities in the ions that constitute the salt. This characteristic determines that the components of a reactive mixture react differently in different solvents, i.e. the ionic liquid functionality determines the overcome of the chemistry reaction and leads to different products.

The acid and the base used for the synthesis of the ionic liquid are, at the same concentration range of the ionic liquid, normally inhibitory of the growth of the microorganism and when growth is still observed the formation of new metabolites was not detected.

The presence of the ionic solvent alters the profile of the products synthesized during growth, and is different from the effect caused in the same culture when in the presence of equal concentrations of a common salt (and ionic strength). The effect becomes stronger for more hydrophobic ions (less water soluble), and apparently not existing a. correlation between the fungal species, or the taxa, and the minimal inhibitory concentration (MIC) of the ionic liquid.

The ability of the ionic liquid to alter the profile of metabolites synthesized during fungi growth results essentially from: i) the solvent functionality determines the extension of the biochemistry reactions leading to the formation of distinct products (the biocatalysis is mediated by extracellular enzymes, cell wall enzymes, or, in the cases where the solvent is absorbed by the cells, by intracellular enzymes); ii) the ions that constitute the ionic liquid are used as precursors for the synthesis of new fungal metabolites, being it possible to distinguish between these two hypotheses analyzing the biodegradability of the solvent.

The productive ability of the fungal biocatalysis also depends on the bioavailability of the substrate, therefore the solubility of the substrate in the catalysis solvent is a crucial factor. The ionic liquids present excellent solvency properties, allowing the present invention to exploit a broad range of substrates, such as the plant water insoluble composites, which are composed by structural and chemical complex polymers (for e.g. suberin, cutin and lignin) and may therefore be used for the biosynthesis of new molecules. The present invention exploits this feature in the fungal biocatalysis which is also useful for inducing, ubiquitously, the synthesis of new fungal metabolites.

In all the tested cases where growth of the fungi mycelium was observed, new metabolites were synthesized.

The natural products diversity hides innumerous pharmacological properties, therefore, fungal biocatalysis in an ionic liquid constitutes a significant advance for the green, non-polluting, synthesis of novel natural products with biological activity. Moreover, this process can also be used for any process where fungal digestion (degradation) of a composite (water soluble, insoluble or poorly soluble) is aimed, because the ionic liquid solubilised composite can be more easily degraded by the filamentous fungi, which becomes especially interesting for use in fungi bioremediation of toxic recalcitrant compounds.

The ionic solvent is solely constituted by ions and therefore these liquid salts do not present measurable vapor pressure, and can be considered green solvents. Consequently, they can be used not only in the pharmaceutical industry, for the synthesis of new pharmacological molecules, but also for the production of already existing pharmaceutics, replacing by this non-polluting technology some dirtier production processes.

They can also be used in the industry of waste management for elimination of solid residues (toxic or not), thus exploiting, concomitantly, the degradation ability of filamentous fungi (even of organic persistent pollutants) with the high solvency of the ionic solvents.

Apart from all mentioned applications, the present invention can still be used in the production of add-valued products obtained through efficient digestion of the food-industry wastes (often enriched in lignin, suberin, cutin and cellulose).

The major advantage of using living-organisms is that instead of restricting the biocatalysis to the plain use of pure enzymes and/or enzyme mixtures, it focuses on the exponential enhancement of the metabolite diversity that fungi are able to synthesize and on uncountable combinations which are possible between the fungi species and the ionic liquid (and/or the substrate).

### Detailed description of the invention

### 1. Culture media

For developing the present invention a poor semi-synthetic media (minimal media, pH 6) is used as culture media containing per liter of water 0,3 g NaNO₄; 0,05 g of KCl; 0,05 g of MgSO₄.7H₂O; 0,001 g of FeSO₄.7H₂O; 0,01 g of ZnSO₄.5H₂O; 0,0005 g of CuSO₄.5H₂O, 1 g of K₂HPO₄ supplemented with 1% glucose (or any other carbon source, such as cork powder, which is extremely rich in suberin and lignin), although the formulation of the media can be different provided that it allows the growth of selected fungi.

The ionic liquid is added to the media at the desired concentration. The mixture is sterilized by filtration.

### 2. Ionic liquids

The ionic liquids can be synthesized from the ions listed in table 1, although not exclusively, and can be used in the culture media with a ionic strength which varies from 0 to 10 M.

### 3. Fungal culture

The growth media containing the ionic solvent is inoculated with a spore suspension, or selected fungus multiplication material.

The spores are collected from a 3 week-aged culture grown in solid media (minimal media jellified with agar 15 g/L). The spores are harvested with a solution of serum (0,85% NaCl)containing 0,1 % Polyoxyethylene sorbitan monolaurate (v/v).

The spore suspension is clarified by filtration throughout sterile glass wool. The spores are recovered by centrifugation (Sigma centrifuge 4K15 with rotor 12166-H, 13500 rpm, 20 minutes at 4 °C) and washed with physiological serum (0,85% de NaCl)containing 10% glycerol (v/v). The spore density in the suspension is quantified by direct counting using a hemocytometer (BH2-RFCA, Olympus).

For screening initially the fungi tolerance to each of the ionic solvents tested, cultures of 250 µL cultivated in 96 wells microplates are used. Consequently, the effect on the fungi growth rate by increasing the ionic solvent concentration can be easily monitored at 600 nm using a spectrometer for microplates.

The growth of the culture (spore germination and mycelium development) is monitored indirectly by the increase of the optical density of the culture media, and/or directly by direct observation of the culture using a magnifying lens or a microscope.

When the culture initiates a new cycle of sporulation the culture extract is collected and the mycelium and spores are removed for analysis. The water in the culture extract can be easily eliminated by liophilisation.

### 4. Analysis of the formed metabolites

The fungi metabolites produced during growth can be extracted from the culture extract using an organic solvent immiscible in the ionic liquid used to supplement the cultured media, such as hexane ethyl acetate or methanol.

The diversity of fungi metabolites extracted from the culture media containing the ionic liquid is analyzed and compared with the metabolites present in the extracts obtained when the fungi is grown in the same media but which contains the same concentration of NaCl, or other common salt, instead of the ionic liquid.

This analysis can be done by any analytical method that allows a good resolution of the components of the mixture, such as liquid chromatography by diode-array or mass spectrometry.

When novel molecules (or new mass) are detected they can be isolated and identify by standard chemical identification methods.

### Examples

### Example 1

A fungi isolate, *Penicillium olsonii,* was used as living organism for screening the effect of different ionic liquids in the production of fungal metabolites during growth.

As culture media a poor semi-synthetic media containing 1% glucose was used (minimal media pH 6) which contains 0,3 g NaNO₄ per liter of medium; 0,05 g of KC1; 0,05 g of MgSO₄.7H₂O; 0,001 g of FeSO₄.7H₂O; 0,01 g of ZnSO₄.5H₂O; 0,0005 g of CuSO₄.5H₂O, 1 g of K₂HPO₄ (the media formulation can be different).

The culture media was supplemented with 0,05 M of each ionic liquid being tested (the maximum inhibitory concentration can be previously determined). The mixture is sterilized by filtration.

As an initial test (example) the following ionic liquids were used: ethyl-methylimidazolium chloride (C₂mimCl), Butyl-methylimidazolium chloride (C₄mimCl), cholinium chloride (ChCl), Methyl-Butylpyrrolidinium chloride (MBPyrrCl), Butyl-pyridinium chloride (C₄PyCl), Ethyl-Methylimidazolium Thiocyanate (C₂mimSCN); Ethyl-Methylimidazolium Ethylsulfate (C₂mimC₂H₅SO₄).

The culture media containing 0,05M of ionic solvent was inoculated with a spore's suspension of *P. olsonii* (10⁵ spores per mL) and the micro-cultures (96 wells microplates) were used for monitoring fungi growth.

The growth behavior of the culture (spores germination and fungal mycelium development) was indirectly measured following an increase in the optical density in the media (600 nm),being confirmed by direct observation under a magnifying glass.

The incubation conditions were kept constant during all growth period and until the culture reaches the stationary phase of growth (~2 weeks after inoculation). The incubation conditions should be near those found optimal for fungi growth, in this case, conditions were: 27°C, in the dark dark and under orbital agitation (90 rpm).

The composition of the filtrated culture extract, i.e. the diversity of fungal metabolites, was analyzed by ESI-MS and compared with the culture extract composition of the same fungal culture when grown in the same media but ionic liquid free, containing or not NaCl. The effect in the culture growth and productive behavior caused by the acid and the base used in the synthesis of the different solvents was also screened.

When the culture reaches the stationary growth phase, the culture extract is recovered. The mycelium and the spores are eliminated by centrifugation and the cellular remains and debris are eliminated by filtration throughout filters of 0,22 µm. The extract can be directly analyzed by liquid chromatography, or, by mass spectrometry, or can be previously concentrated by liophilisation, before or after the ionic liquid elimination by metabolites extraction using and organic solvent immiscible in the ionic liquid (in this case ethyl acetate).

In the following table, the new mass species of fungi origin which were produced specifically in the presence of the ionic solvent (relatively to the metabolites produced in the presence of the common salt) are indicated (detected in the negative mode ESI-MS). In this example the extracts used were clarified by liophilisation to eliminate the water. The mass species which are also detected in the controls, i.e. in the culture extracts of the fungi grown in an ionic liquid free media and in the presence of 0,05 M of NaCl, are not considered.

| **P. *olsonii*** | **New mass species** |
|---|---|
| C₂mimCl | 179, 216, 666, 736 |
| C₂mimC₂H₅SO₄ | 210, 273 |
| C₂mimSCN | inhibition of growth |
| MBPyrrCl | 137, 179, 210, 238, 257, 296, 358 |
| C₄PyrCl | 125, 161, 210, 311, 325, 430, 480, 530, 607, 631 |
| ChCl | 179, 216, 456 |

In this specific case amongst the 6 ionic liquids tested only the addition to the culture media of 0,05M of C₂mimSCN was completely inhibitory to the fungi growth. In the remaining ionic liquids the presence of the solvent induced, relatively to the control cultures grown in the presence of NaCl, ubiquitously the formation of novel metabolites, which is demonstrated by the profile of mass species detected by mass spectrometry (ESI). Apparently, the diversity of mass species increases for ionic liquids was enhanced the higher the "hydrophobic" nature of the ionic solvent, i.e. in the presence of ionic liquids very hydrophilic, i.e. extremely soluble in water: C₂mimCl, C₂mimC₂H₅SO₄ and ChCl, the number of metabolites is lower than in the case of less water soluble ones: C₄PyCl and MBPyrrCl. In this example, some of the detected mass species are not specific, i.e., some masses were produced by the same culture in different ionic liquids (underlined mass species).

### Example 2

The fungi isolate, *Trichoderma longibrachiatum,* was used as living organism for screening the effect of different ionic liquids in the production of fungal metabolites during growth.

The media used was the same already described in example 1 which was supplemented with 0,05 M of each ionic liquid being tested. As an initial test (example) the following ionic liquids were used: ethyl-methylimidazolium chloride (C₂mimCl), Butyl-methylimidazolium chloride (C₄mimCl), cholinium chloride (ChCl), Methyl-Butylpyrrolidinium chloride (MBPyrrCl), Butyl-pyridinium chloride (C₄PyCl), Ethyl-Methylimidazolium Thiocyanate (C₂mimSCN); Ethyl-Methylimidazolium Ethylsulfate (C₂mimC₂H₅SO₄).

The culture media containing 0,05M of the ionic solvent was inoculated with a spore's suspension of *T. longibrachiatum* (10⁵ spores per mL) and the micro-cultures (96 wells microplates) were used for monitoring fungi growth. The incubation conditions were kept constant during all growth period and until the culture reaches the stationary phase of growth (~2 weeks after inoculation).

The composition of the filtrated culture extract, i.e. the diversity of fungal metabolites, was analyzed by ESI-MS and compared with the culture extract composition of the same fungal culture when grown in the same media but ionic liquid free, containing or not 0,05 NaCl. The effect in the culture growth and productive behavior caused by the acid and the base used in the synthesis of the different solvents was also screened.

When the culture reaches the stationary growth phase, the culture extract is recovered, clarified and concentrated as described in the example 1. The mass species detected in the controls, i.e., in the fungus culture extract which was grown without ionic solvent and in the presence of 0,05M of NaCl or in ionic liquids are not considered.

In the following table the new mass species of fungi origin which were produced specifically in the presence of the ionic solvent (relatively to the metabolites produced in the presence of the common 0.05 M of salt) are indicated (detected in the negative mode ESI-MS).

| ***T. longibrachiatum*** | **New mass species** |
|---|---|
| C₂mimCl | Growth inhibition |
| C₂mimC₂H₅SO₄ | Growth inhibition |
| C₂mimSCN | Growth inhibition |
| MBPyrrCl | 137, 165, 198, 210, 238, 284, 300, 358, 372, 456, 478 |
| C₄PyCl | Growth inhibition |
| ChCl | 120, 155, 228 |

In this specific case amongst the 6 ionic liquids tested the addition to the culture media of the three ionic liquids containing the cation C₂mim, such as C₂mimCl, C₂mimC₂H₅SO₄, and C₂mimSCN, were inhibitory to the fungi growth. The ionic liquid C₄PyCl also inhibited, at the tested concentration, the development of the mycelium. In the remaining ionic liquids the presence of the solvent has induced, relatively to the control cultures grown in the presence of a NaCl, ubiquitously, the formation of novel metabolites, which is demonstrated by the profile of mass species detected by mass spectrometry (ESI). Apparently, the diversity of mass species formed, increase for solvents carrying a higher "hydrophobic" nature of the ion solvent, e.g. in the presence of ChCl the number of new metabolites is lower than in the case of the less water soluble MBPyrrCl. In this example all detected mass species are specific for the combination fungi species and ionic liquid.

### References

1. Oliveira, A, Peres, C, Correia Pires, J, Silva Pereira, C, Vitorino, S, Figueiredo Marques, J, Barreto Crespo, M, and San Romao, M. Cork stoppers industry: defining appropriate mould colonization. Microbiol Res 2003; 158(2): 117-24.
2. Silva Pereira, C, Pires, A, Valle, M, Vilas-Boas, L, Figueiredo Marques, J, and San Romão, M. Role of Chrysonilia sitophila on the quality for cork stoppers for sealing wine bottle. J Ind Microbiol Biotech 2000; (24): 256-261.
3. Silva Pereira, C, Soares, G, Oliveira, A, Rosa, M, Pereira, H, Moreno, N, and Romao, M. Effect of fungal colonization on mechanical performance of cork. Int Biodeter Biodegr 2006; 57(4): 244-250.
4. Basílio, MC, Gaspar, R, Silva Pereira, C, and San Romão, MV. Penicillium glabrum cork colonising isolates - preliminary analysis of their genomic similarity. Rev Iberoam Micol 2006; 23(3): 151-154.
5. Kis-Papo, T, Oren, A, Wasser, SP, and Nevo, E. Survival of Filamentous Fungi in Hypersaline Dead Sea Water. Microb. Ecol. 2003; 45: 183-190.
6. Kis-Papo, T, Kirzhner, V, Wasser, SP, and Nevo, E. Evolution of genomic diversity and sex at extreme environments: Fungal life under hypersaline Dead Sea stress. PNAS 2003; 100(25): 14970-14975.

## Claims

1. Fungal biocatalysis process **characterized in that** a fungi strain is grown in a culture medium which is supplemented with water soluble ionic liquids, comprising the following steps:
a) Addition of an ionic liquid to the culture media which is specifically functionalized for inducing fungi growth;
b) Inoculation of the culture media, supplemented with the ionic liquid, with the fungal inoculum material selected, for example, spores or mycellium;
c) Collection of the metabolites produced by the fungi culture;
d) Analysis of the extracted metabolites.

2. Fungal biocatalysis process, according to claim 1, **characterized in that** the culture media used for growing the fungi is preferably a poor semi-synthetic media containing 1% of a carbon source.

3. Fungal biocatalysis process, according to the preceding claims **characterized in that** the fact that the culture media used for growing the fungi contains a source of lignin, and/or suberin, and/or cutin, for example, cork powder.

4. Fungal biocatalysis process, according to claim 1, **characterized in that** the ionic liquid solubilised in the culture media is composed of one cation and one anion which carry specific organic functionalities, such as those which provide the solvent with a hydrophilic/hydrophobic and/or lipophilic / lipophobic nature.

5. Fungal biocatalysis process, according to the preceding claim, **characterized in that** the liquid ionic strength changes between infinite dilution and 10 M.

6. Fungal biocatalysis process, according to the preceding claims **characterized in that** the culture of the selected fungus may be cultivated in a continuous, semi-continuous or discontinuous mode.

7. Fungal biocatalysis process, according to claim 1, **characterized in that** the collection of the metabolites produced by the fungi in the ionic liquid supplemented media, is done by using an organic solvent which is immiscible in the ionic liquid used, such as ethyl acetate.

8. Use of a fungal biocatalysis process according to the preceding claims, **characterized in that** it is used in the induction of the metabolite synthesis in a fungi culture.

9. Use of a fungal biocatalysis process according to the preceding claims, **characterized in that** it is used in the induction of new fungal metabolites biosynthetic pathways.
